# EUROPEAN PATENT SPECIFICATION

(11) **EP 4 646 186 B1**
(45) Date of publication and mention of the grant of the patent: **29.04.2026**
(21) Application number: 23844198.4
(22) Date of filing: 28.12.2023
(51) Int. Cl.: A61K 9/127, A61K 9/1277, A61K 31/12, A61K 47/12, A61K 47/22

(54) **LIPOSOMAL FORMULATION OF CURCUMIN FOR OPHTHALMIC USE AND ITS INDUSTRIAL PREPARATION METHOD TO STABILISE CURCUMIN CONCENTRATION OVER TIME**
LIPOSOMALE FORMULIERUNG VON CURCUMIN FÜR DIE AUGENHEILKUNDE UND IHRE INDUSTRIELLE HERSTELLUNGSMETHODE ZUR STABILISIERUNG DER CURCUMIN-KONZENTRATION ÜBER EINEN LÄNGEREN ZEITRAUM
FORMULATION LIPOSOMALE DE CURCUMINE POUR USAGE OPHTALMIQUE ET SA MÉTHODE DE PRÉPARATION INDUSTRIELLE POUR STABILISER LA CONCENTRATION DE CURCUMINE DANS LE TEMPS

(30) Priority: 02.01.2023 IT 202300000006
(43) Date of publication of application: 12.11.2025
(73) Proprietor: Offhealth S.p.A., 50144 Firenze (FI) (IT)
(72) Inventor: ZANINI, Alessandro, 56011 Calci (PI) (IT); VELLANTE, Marco, 62029 Tolentino (MC) (IT); SODO, Eugenio, 00125 Rome (RM) (IT); CAVALLO, Giovanni, 00122 Roma Ostia (RM) (IT); FOSCHINI, Fulvio, 00124 Rome (RM) (IT); GIUVA, Roberto, 62012 Civitanova Marche (MC) (IT)
(74) Representative: Sarpi, Maurizio
(86) International application number: PCT/IB2023/063312
(87) International publication number: WO 2024/147064

(56) References cited:
- WO-A1-2021/044283
- CN-A- 102 379 850

## Description

The present invention relates to a new liposomal formulation based on curcumin for ophthalmic use, and the preparation method thereof aimed at stabilizing the concentration of curcumin itself over time, overcoming the limits of the formulation covered by Italian patent no. 102019000005408 to the same Applicant, which especially showed a decrease in the titer of curcumin over time.

In the cited patent, there is claimed an amount of curcumin between 0.025% and 0.050% by weight, on the total weight of the formulation, so that such a formulation is filterable and sterilizable at 0.2 microns, and the determining presence of vitamin E-D-α-tocopheryl polyethylene glycol (E-TPGS) with the dual purpose of promoting the formation and filterability of liposomes while providing, at least as a theoretical expectation, a general stability to the formulation, expressed by the constant concentration of curcumin over time.

In particular, the amount of Vitamin E TPGS is between 0.05 and 0.5% by weight, with respect to the weight of the final composition, which values are below the ocular cytotoxicity threshold which is recognized in the literature based on scientific evidence and which lead to a solubilizing effect of Curcumin, overcoming the critical issues present to date in eye drops based on curcumin-based liposome formulations.

In addition to the above statements, with a concentration above 0.5% by weight, vitamin E-TGPS can have a cytotoxic effect, being a surfactant (irritant) at the ocular level, as can be seen on the other hand from the products currently on the market and from the literature. And this limits the use thereof at an ophthalmic level.

Therefore, a formulation has been experimentally defined where, only with a defined range of concentrations, vitamin E-TPGS is compatible with the ocular use thereof, leaving the liposomes the primary role of carrier to convey the curcumin, and enhancing, at least theoretically with reference to the presence of vitamin E TPGS, the antioxidant effect thereof: specifically, the best ratio of Vitamin E-TPGS and Curcumin is 1/1 by weight and the ratio of Vitamin E-TPGS and phospholipids is 1/20 by weight (0.05% Vitamin E-TPGS / 1% Phospholipids).

Following the checks carried out on the first batches produced industrially, however, it was found that curcumin (stable in eye drop vial) is subject to degradation which is accentuated with temperatures above 25°C, which strongly limits the marketing thereof.

It is thus necessary to overcome such problems.

Therefore, it is an object of the present invention to provide a liposomal formulation with curcumin for ophthalmic use, and a method for the production thereof, which leads to the production of a stable product over time, in a range between 4°C and 40°C, thus allowing the eye drops to be marketed for 18-24 months at room temperature.

This was achieved by modifying the production method covered by Italian patent 102019000005408 to the same Applicant, which is characterized by the following operating steps:
a) Solubilization in organic solvent of non-hydrogenated phospholipids with curcumin (specific inclusion of the active ingredient in the phospholipids);
b) vacuum-drying the organic mixture consisting of phospholipids and curcumin until the solvents are completely eliminated;
c) hydration in water of the dry phospholipid paste (aqueous solution containing the buffer and other excipients) for an appropriate hydration time;
d) subsequent homogenization with a shear homogenizer for an appropriate period of time to obtain a mixture of Pro liposomes;
e) extrusion of the Pro liposome solution at high pressure 900-1000 atm for at least 5 times to obtain the desired *size* less than 200 nm;
f) 0.2 micron in-line filtration of the just-extruded liposome solution;
   and lastly
g) collection of the liposome solution sterilized by 0.2 micron filtration in a sterile container.

According to the present invention, the two operating steps are modified:
- vacuum-drying step (b), and
- 0.2 micron filtration step (f).

In particular:
the vacuum-drying step was modified by specifically adding antioxidants/anti-lipophilic radicals, such as BHA,
Ascorbyl palmitate and ferulic acid, to the starting alcohol solution containing the Phospholipids, Curcumin and Vitamin E TPGS.

This change arises from the following considerations:
First consideration:
   As reported in the literature, (see References, ref. 1, 1a), curcumin degrades easily, as it interacts with oxygen (and more generally with oxidants) and tends to hydrolyze over time.
Second consideration:
   The presence of the Vitamin E TPGS alone, in the drying step, is not sufficient, despite the above statements, to carry out an antioxidant effect. On the other hand, to be truly effective as an antioxidant, it needs to be in free and unesterified form (ref. 2).

Therefore, it must be hydrolyzed: this occurs due to an Enzyme such as a Hydrolase (Esterase) which is present only in vivo on the mucous membranes/tissues, and not during a drying process, also considering the fact that Vitamin E TPGS is particularly stable even at high temperature and thus cannot be in free form and thus active as an antioxidant.

### Third consideration

With the intent to protect curcumin, a specific mixture of antioxidants has been selected such as BHA (Butylhydroxyanisole) and Ascorbyl palmitate which, being lipophilic, interact directly with the phospholipids and curcumin: ferulic acid has been added to these two lipophilic antioxidants, which, in addition to being an excellent lipophilic antioxidant, is a product (although not the majority) of the curcumin hydrolysis reaction, whereby the presence thereof can inhibit, or in any case slow down, at the chemical equilibrium level, the hydrolysis reaction itself (ref. 3).

The 0.2 micron filtration step was instead modified by adding, before filtration, sodium ascorbate to the aqueous solution of freshly extruded liposomes, which is a reducing agent, or rather an agent "capable of almost completely removing oxygen dissolved in a buffered solution" (see ref. 4), specifically liposomal, so as to increase, as experimentally demonstrated, the stabilization of the Curcumin itself.

Further features and advantages of the present invention will become apparent from the following detailed description, referring to the accompanying drawings which show:

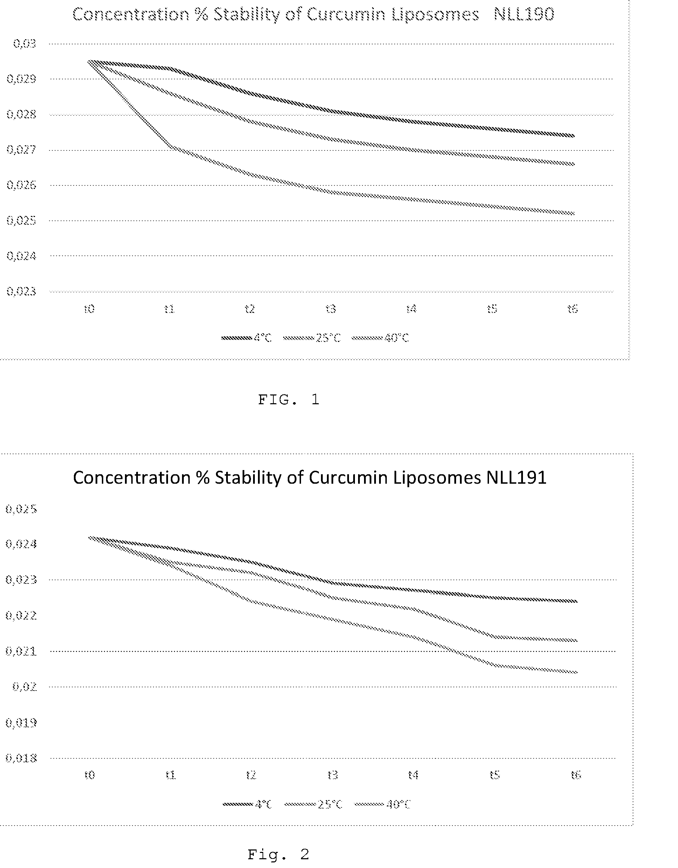

Figure 1 is a graph showing the stability of non-hydrogenated phospholipid liposomes containing Curcumin, Vit. E TPGS, Ferulic Acid intercalated in the liposomal structure, with sodium ascorbate added outside the liposomes after extrusion and before filtration, at the different temperatures 4°C, 25°C and 40°C, where the result at 40°C is comparable to 24 months of room temperature; (preparation NLL-190)
Figure 2 is the graph of the stability of the liposomes of hydrogenated phospholipids containing Curcumin with Ferulic Acid, BHA, Vitamin E TPGS and Sodium Ascorbate, added in the extrusion step of the hydrated phospholipid mixture, as well as, later, in the 0.2 micron filtration step of the aqueous solution, outside the liposomes, at the different temperatures 4.25 and 40 °C. ( preparation NLL-191)

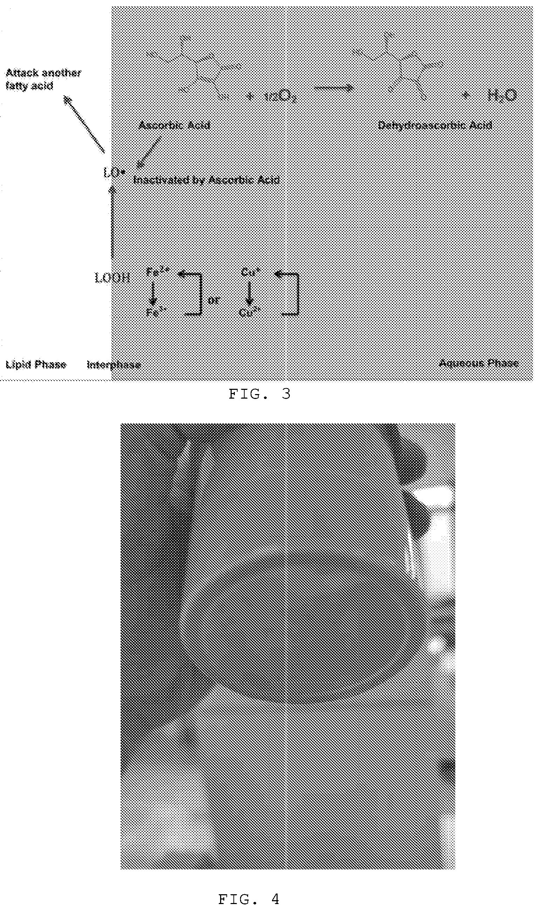

Figure 3 shows how ascorbic acid is capable of almost completely removing oxygen dissolved in a buffered solution.
Figure 4 shows in the photograph how with the product obtained according to the FAST 1 method, as well as that obtained according to the FAST 2 method, where the drying step is not included, the liposomes extruded after a couple of hours from the extrusion always tend to sediment.

### CURCUMIN LIPOSOME FORMULA REVISION

With reference to the above description, we started from the formulation of Italian Patent No. 102019000005408 to the same Applicant, in which the following were present:

### Curcumin Liposomes 0.05%/Vitamin E TPGS 0.05% with HYDROGENATED phospholipids.

| Raw materials | %w/w | CAS number |
|---|---|---|
| Curcumin | 0.040 | 458-37-7 |
| Phospholipids 90H | 1.000 | - |
| Vitamin E TPGS | 0.050 | 9002-96-4 |
| Boric acid | 0.775 | 10043-35-3 |
| SODIUM TETRABORATE DECAHYDRATE | 0.125 | 1303-96-4 |
| Sodium chloride | 0.450 | 7647-14-5 |
| Distilled water as needed to | | - |
| 0.2 micron sterile filtration | yes | - |

The industrial production (only 30-40 liters) of the formula above indicated an unexpected decrease in the curcumin titer and also a poor stability over time, as can be seen from the data reported in the table below.

### Formulation stability

| Time Months | 4°C %w/w | 25°C %w/w | 40°C %w/w |
|---|---|---|---|
| 0 | 0.0210 | 0.0210 | 0.0210 |
| 1 | 0.0150 | 0.0100 | 0.0020 |
| Decrease % | 28.58 | 52.39 | 90.5 |
| Residual amount % | 71.42 | 47.61 | 9.5 |

### Stability data review

These data drew the attention of the inventors who noted that the inclusion of curcumin in liposomes, consisting of hydrogenated phospholipids, theoretically more stable/compact, was not sufficient to ensure a good stability of the molecule over time.

The degradation phenomenon was rationalized /interpreted as a confirmation that curcumin is known to be a particularly unstable molecule which tends to degrade over time.

### Preparation data review

Compared to the theoretical titer 0.040%, it was verified that the actual Titer is equal to 0.0210%.

The loss during the preparation, extrusion and subsequent sterilization step by means of 0.2 micron filtration is therefore equal to 47.5%.

In an attempt to solve the various problems related to both the stability over time and the actual titer of curcumin, lower than the theoretical titer already at Time 0, further tests and productions were carried out. A formula is show below relating in particular the use of non-hydrogenated Phospholipids and the addition of Ferulic acid (Ref. 3).

### Formulation:

### NLL-182A: Curcumin Liposomes (Phospholipids S80)

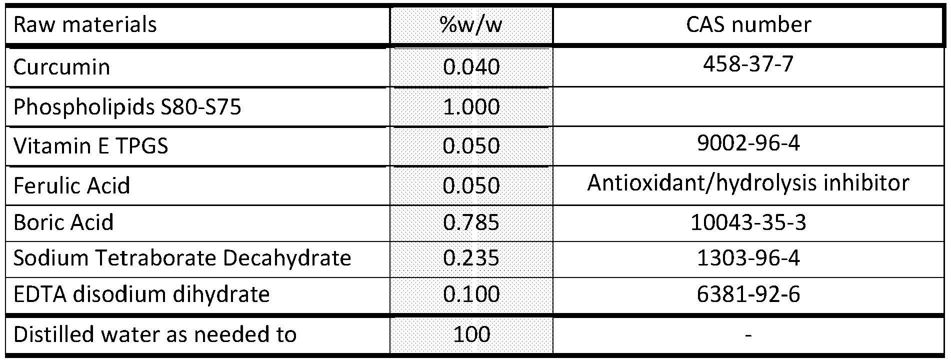

### Formulation stability:

| Time Months | 4°C %w/w | 25°C %w/w | 40°C %w/w |
|---|---|---|---|
| 0 | 0.0239 | 0.0239 | 0.0239 |
| 1 | 0.0167 | 0.0155 | 0.0114 |
| Decrease % | 30.13 | 35.15 | 52.31 |
| Residual amount % | 69.87 | 64.85 | 47.69 |

### Stability data review

It is interesting to note that the presence of Ferulic acid had a certain effect on the curcumin titer at different temperatures, but was not sufficient to ensure a prolonged stability of at least 1 year (3 months at 40°C)

The addition of ferulic acid, in addition to having an antioxidant effect, as indicated above, was done for the purpose of reducing the hydrolysis reaction of curcumin which leads to the formation of various compounds including, indeed, ferulic acid.

In other words, the presence thereof in solution should, at least in part, condition/slow down the hydrolysis reaction, forcing the equilibrium towards curcumin.

However, the antioxidant/anti-radical capacity of ferulic acid was not sufficient, because already after the first month of stability at 40°C, the concentration of curcumin decreased by 52% with respect to the initial value, thus not ensuring a stability of at least 18-24 months at room temperature.

### Preparation data review

- Theoretical titer 0.040%; Actual titer: 0.0239%
- Loss in the preparation and filtration step: 40.25%.

The decrease in titer can be attributable to the instability of Curcumin during extrusion and subsequent 0.2 micron filtration of the liposomal curcumin solution.

Assuming that the degradation of curcumin was nonspecifically linked to a possible effect of light, in the production, drying and extrusion step, it was thought to add Vitamin B12 to the formulation indicated above, with the intention that it could act as a screen for light.

Therefore, the following formulation was developed and subjected to stability tests:

### NLL-182B: Curcumin Liposomes (Phospholipids S80+B12)

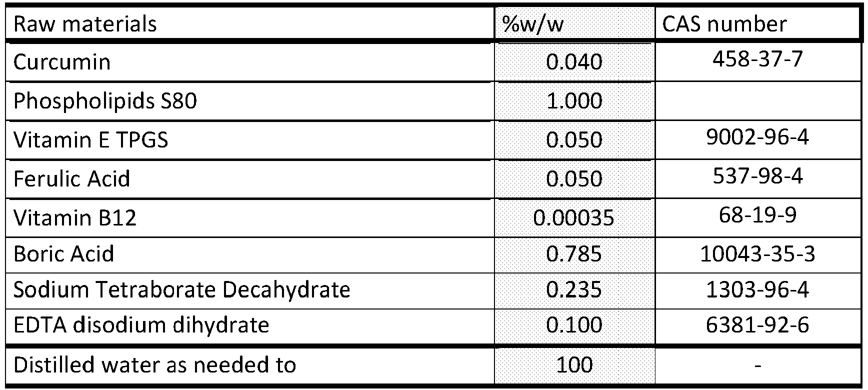

which provided the following results:

### Formulation stability

| Time Months | 4°C % | 25°C | 40°C |
|---|---|---|---|
| 0 | 0.0212 | 0.0212 | 0.0212 |
| 1 | 0.0154 | 0.0141 | 0.0120 |
| Decrease% | 27.37 | 33.5 | 43.40 |
| Residual amount % | 72.63 | 65.5 | 56.60 |

### - Stability data review

Interestingly, the presence of ferulic acid with the addition of Vitamin b12 did not change the ability to stabilize the product over time: in fact, the residual amount of curcumin, after 1 month at 40°C, was 56.60.

### - Preparation data review

Theoretical titer 0.040%,
Actual Titer: 0.0212%.

The loss during the preparation and filtration step is 47%, i.e., slightly increased.

Assuming that the degradation of curcumin was linked to the need to have a strongly reducing environment so as not to favor the possible oxidation of Curcumin, it was thought to prepare a formulation with Monothioglycerol, recognized to be a powerful reductant. (Ref. 4)

### NLL-185: Curcumin Liposomes (Phospholipids S80 MonoThioglycerol)

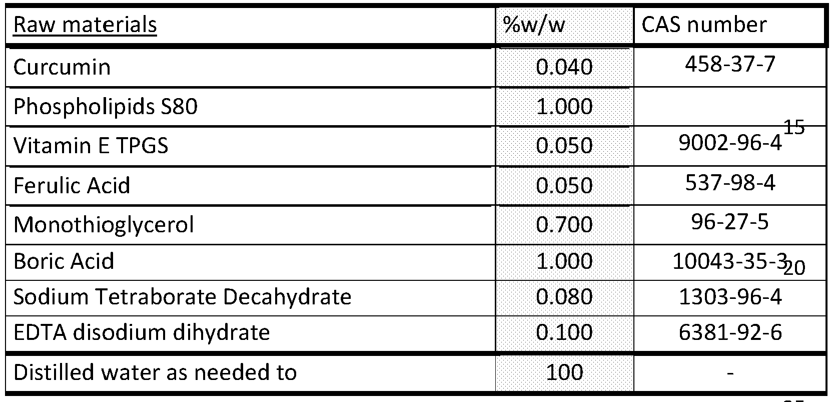

### Formulation stability

| Time Months | 40°C |
|---|---|
| 0 | 0.0088 |
| 1 | 0.0057 |
| Decrease% | 78 |

### - Review of stability data

However, the presence of ferulic acid and thioglycerol did not lead to any improvement in stability. Indeed, the assumption that a reducing environment could be a stabilizing factor was erroneous.

### - Preparation data review:

The theoretical titer of 0.040% decreased drastically, by 78%. The reducing feature of Thioglycerol does not seem to have an effect of reducing the degradation of Curcumin, on the contrary it accelerates it. The above results led the Inventors to consider the possibility that Curcumin, although inserted in the liposomal structure, could interact the same over time with the dissolved oxygen in the aqueous medium, even in the presence of an antioxidant, such as vitamin E TPGS, Ferulic Acid, and that the presence of a strong reductant, such as Thioglycerol, was not the most appropriate solution and therefore it was necessary to identify another molecule which could reduce the presence of dissolved oxygen in water. In this respect, the following formulation was developed, where in particular Sodium Ascorbate was added to the solution of freshly extruded liposomes in consideration of the antioxidant capacity thereof and the solubility thereof in water, (Ref. 5), obtaining the following formulation (it should be noted that sodium ascorbate is outside the liposomes).

### NLL-184: Curcumin Liposomes (Phospholipids S80+ Sodium Ascorbate) 25-liter preparation using a 5-inch filter (0.8-0.2 microns)

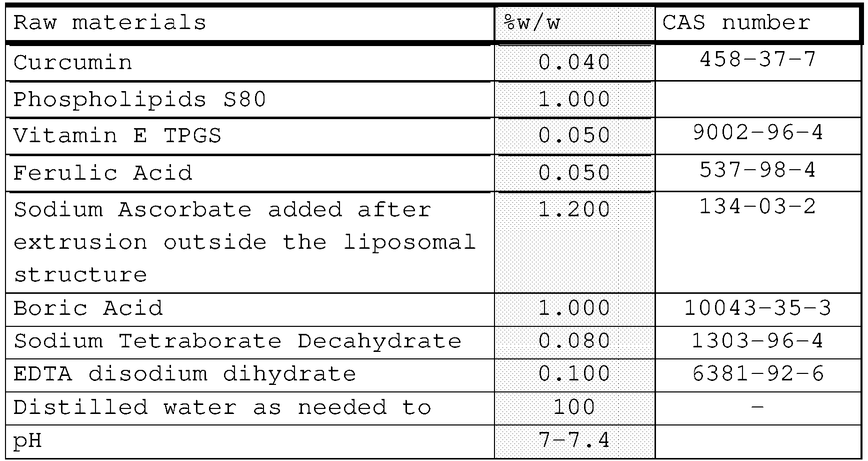

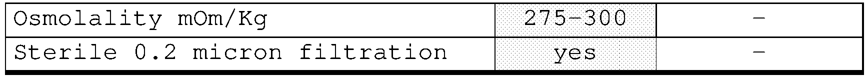

Filtration proceeded steadily at a rate of 100ml/minute.

The checks carried out gave the following results:

### Formulation stability

| Time Months | 40°C |
|---|---|
| 0 | 0.0224 |
| 1 | 0.0197 |
| Decrease% | 12.06 |
| Residual amount % | 87.94 |

### Stability data review

The specific presence of ferulic acid and sodium ascorbate led to an improvement in stability at 40°C even if evaluated for only one month. Vitamin E TPGS certainly did not affect the stability over time because it has no antioxidant effect, being esterified.

### Preparation data review

The theoretical titer of 0.040% decreased by 44%.

This result, encouraging in terms of stability, is however negative due to the 44% reduction in the theoretical curcumin titer. This led the inventors to review the various steps forming the entire production process.

Below are the various steps:
- First step: Solubilization of phospholipids, curcumin and vitamin E TPGS and ferulic acid with ethyl alcohol;
- Second step: Vacuum-drying of the alcohol solution to obtain a dry phospholipid powder;
- Third step: Dispersion of the phospholipid powder in buffer;

- Fourth step: High-pressure extrusion for a certain number of times to obtain the liposomes;
- Fifth step: pH control, osmolality and 0.2 micron sterilizing filtration in sterile bag.

In particular, the inventors reviewed the First and the Second Step which are respectively: Solubilization and Vacuum-drying.
- In the First Step, the alcohol solution containing the Phospholipids, Curcumin, Vitamin E TPGS and Ferulic Acid, and consisting of lipophilic molecules, was vacuum-dried at controlled temperature.
- Drying under vacuum at a controlled temperature of 40°C is certainly a step which subjects Curcumin to the possibility of being degraded due to the temperature and the presence of oxygen.

To minimize this possible danger and further improve stability over time, two specific LIPOPHILIC antioxidants were added:
3-tert-butyl-4-hydroxyanisole (BHA) and Ascorbyl palmitate with lipophilic features.

BHA is a lipophilic substance and therefore has the peculiarity of carrying out the anti-radical/antioxidant action in lipophilic products (districts), (Ref 6), thus it has been assumed that it could be particularly useful in drying the mixture containing Phospholipids, Curcumin, Vitamin E TPGS and Ferulic Acid, inserting itself inside the lipophilic film. Ascorbyl Palmitate, a lipophilic molecule, has an antioxidant/anti-radical effect very often used in emulsions: in the structure of liposomes, the lipophilic tail thereof (palmitic acid) is intercalated in the phospholipid layer, while the hydrophilic end thereof, ascorbate, is instead positioned outside the liposomal structure. The simultaneous presence of BDA and Ascorbyl palmitate is justified by the fact that the first molecule is inside the liposomal structure (lipophilic), while the second molecule has the polar head (the Ascorbate radical) outside the liposomes and the tail is anchored to the phospholipid film, thus the two molecules carry out the antioxidant function thereof both inside and outside the liposomal structure once the latter is formed by the effect of extrusion.

### NLL-188: Curcumin Liposomes (Phospholipids S80) 50 LITER PREPARATION USING A 10 INCH FILTER (0.8-0.2 MICRONS)

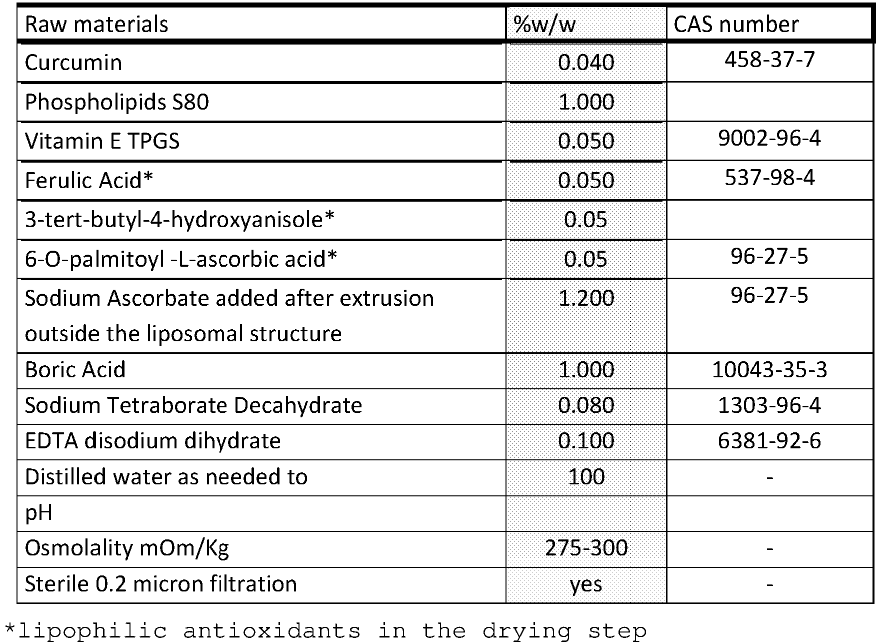

The Filtration went very well: with a rate of 150 ml/minute, the use of a 10-inch filter improved filterability. This parameter, the size of the filtering surface, is important at an industrial level above all, to reduce the time required to filter volumes of 50-10 liters.

### Preparation data review

From an initial curcumin titer of 0.040%, the titer after filtration was 0.0262%: the titer therefore decreased by 34.5%.

It has thus passed from a decrease of 44% in the previous formulation NLL 184, to 34.5% in the formulation NLL 188.

This result indicates that the addition of BHA, Ascorbyl Palmitate, Vitamin E TPGS and Ferulic Acid in the drying step and the addition of Sodium Ascorbate before filtration, improve the final yield of the product.

Considering this preliminary result, the inventors:
increased the size of the filter for filtration (0.8-0.2 microns), which passed from 10 inches to 20 inches;
increased the concentration of Vitamin E TPGS, which passed from 0.04% to 0.4% to make the liposomes less viscous and more fluid, in order to increase the filtration rate, while maintaining the concentration of BHA at 0.05%, Ascorbyl Palmitate at 0.050% and Ferulic acid at 0.050%.

The formulation developed is as follows:
Formulation NLL-190: Curcumin Liposomes (Vitamin E TPGS 0.4% + Sodium Ascorbate)- 50 liter preparation, using a 20 inch filter (0.8-0.2 microns). (FILTRATION RATE)

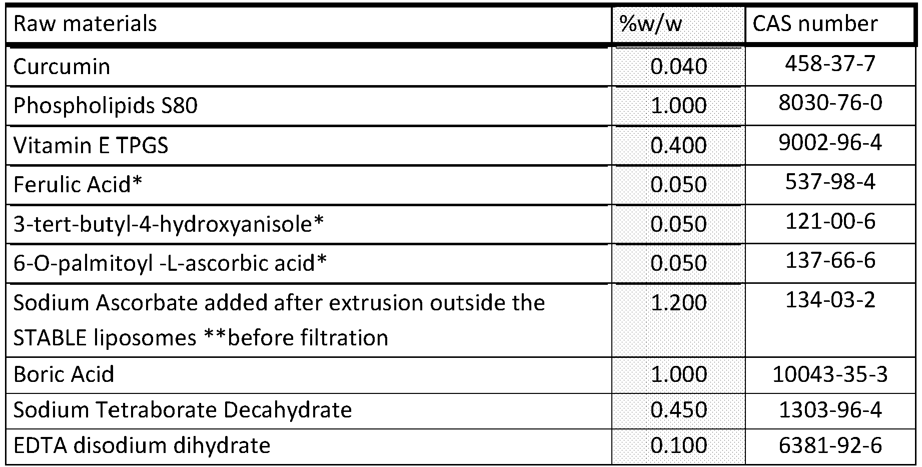

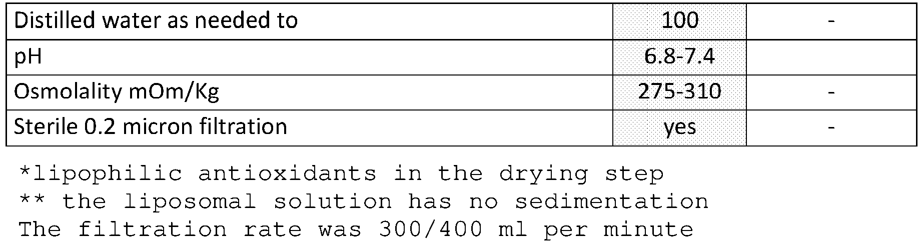

| PREPARATION DATA | % | *Loss % |
|---|---|---|
| Curcumin theoretical value before extrusion | 0.040 | |
| Titer of Curcumin in liposomes after extrusion | 0.0346 | 13.5 |
| Titer of Curcumin in liposomes after filtration | 0.0295 | 26.25 |

| | | |
|---|---|---|
| *Loss % with respect to theoretical | | |

### Preparation data review

The titer of curcumin after extrusion is 0.0346%, with a decrease of 13.5% with respect to the initial value of 0.04%: this is an excellent result which shows how the drying of non-hydrogenated Phospholipids + Curcumin, with the addition of 0.05% BHA, 0.05% Ascorbyl Palmitate, and 0.05% Ferulic Acid, leads to a more stable product or rather with a higher concentration of curcumin also due to the higher concentration of vitamin TPGS, which improves the filterability of liposomes, but which has no antioxidant effect because it is esterified.

The finished product, preparation NLL-190 consisting of the curcumin liposomes, to which sodium ascorbate was added in the aqueous phase after high-pressure extrusion, and which were then sterilized by 0.2 micron filtration, has a curcumin titer of 0.0295%, with a decrease of 26.25%, calculated on the theoretical titer of 0.04%.

This result demonstrates how the addition of sodium ascorbate stabilizes curcumin during filtration; moreover, the presence of the Vitamin E TPGS, with the use of a 20-inch filter, improves the filterability of the product.

This is the best result obtained with respect to all the formulations prepared and reported in the present disclosure.

### Summary

| | |
|---|---|
| Drying step | Phospholipids+ Curcumin + BHA, Ascorbyl Palmitate + Vit. E TPGS |
| Liposome Preparation: High-pressure extrusion | Phospholipid mixture in Buffer |
| Mixing | Addition of Sodium ascorbate to the solution of ALREADY FORMED liposomes |
| Sterilization | By means of 0.2µ Filtration |
| | Sterile product in stability |

The product was divided into the usual eye drop vials and stabilized.

| STABILITY | 4°C | *Degr. % | 25°C | Degr. % | 40°C* | Degr.% |
|---|---|---|---|---|---|---|
| Stability | % w/w | | %w/w | %w/w | % w/W | % w/W |
| To | 0.0295 | 0 | 0.0295 | 0 | 0.0295 | 0 |
| T1 | 0.0293 | 0.6 | 0.0286 | 3.06 | 0.0271 | 8.04 |
| T2 | 0.0286 | 3.06 | 0.0278 | 5.77 | 0.0263 | 10.85 |
| T3 | 0.0281 | 4.74 | 0.0273 | 07.46 | 0.0258 | 12.55 |
| T4 | 0.0278 | 5.77 | 0.0270 | 8.48 | 0.0256 | 13.23 |
| T5 | 0.0276 | 6.45 | 0.0268 | 9.16 | 0.0254 | 13.9 |
| T6 | 0.0274 | 7.12 | 0.0266 | 9.83 | 0.0252 | 14.58 |
| | | | | | | |
| Degradation % | | 7.12 | | 9.83 | | 14.58 |
| | | | | | | |

| | | | | | | |
|---|---|---|---|---|---|---|
| *Stability at 40°C for six months is equivalent to 24 months at room temperature . | | | | | | |

### Stability data review

The presence of Ferulic acid, BHA, Ascorbyl palmitate, and Vitamin E TPGS, intercalated in the liposomal structure, because added to the phospholipids and curcumin in the drying step, and the subsequent addition of Sodium Ascorbate outside the liposomes, just extruded, leads to a considerable improvement in the stability of Curcumin, at all temperatures 4°C, 25°C and 40°C.

In particular, the result at 40°C is comparable to 24 months at room temperature.

### CONCLUSION

Reviewing the production process in the various steps, it can be concluded that:
1) the preparation of curcumin liposomes must take into account the stabilization of curcumin during the drying step: the particular antioxidants/lipophilic anti-radicals in this step contribute to the final stability (over time). It should be specified again that Vitamin E TPGS has no effect in this event, since it has no antioxidant effect, being esterified and thus inactive, it only improves filterability.
2) The addition of Sodium Ascorbate outside the liposomes, just extruded, in the buffer solution, before filtration, improves the final titer of curcumin and increases the stabilization of Curcumin even during the period of stability at different temperatures.

This result indicates that the presence of acidic sodium ascorbate outside the liposomes could have reduced the presence of dissolved oxygen in the liposomal solution, Fig. 1.

In particular, the trend of stability over time indicates a greater decrease in the Curcumin titer, at the beginning of stability. Subsequently, the decrease is less rapid, and this can be interpreted as the fact that ascorbate reacts with oxygen slowly and at the beginning, when the oxygen concentration is higher, it fails to have a high (RAPID) sequestering capacity.

3) The addition of Vitamin E TPGS, and the use of a 20-inch filter, improves the filterability of liposomes, which have a size of less than 200 nm, but does not affect the prolonged stability over time.

Such positive results, which derive from the addition of sodium ascorbate to the just-extruded liposomes and the subsequent 0.2 micron filtration of the entire preparation, prompted the inventors to evaluate/test a procedure which includes:
the addition of a 1^{st} aliquot of sodium ascorbate to the phospholipid powder dispersed in buffer (containing the lipophilic antioxidants, BHA, Ascorbyl Palmitate, Ferulic Acid, Vitamin E TPGS and Curcumin); and
subsequent high-pressure extrusion to form the liposomes, and
the addition to the same liposomes of a 2^{nd} aliquot of sodium ascorbate prior to 0.2 micron filtration.

This procedure derives from the assumption, made by the inventors, that during high-pressure extrusion a degradation of curcumin can occur, due to the presence of oxygen in the solution and the increase in temperature.

Therefore the following formulation was prepared, which also has the particularity of having Hydrogenated Phospholipids (thus in theory with a greater ability to stabilize Curcumin).

### Formulation NLL-191:

### Curcumin Liposomes (Vitamin E TPGS 0.4% + Sodium Ascorbate) 50 liter preparation, use of a 20 inch filter (0.8-0.2 microns)

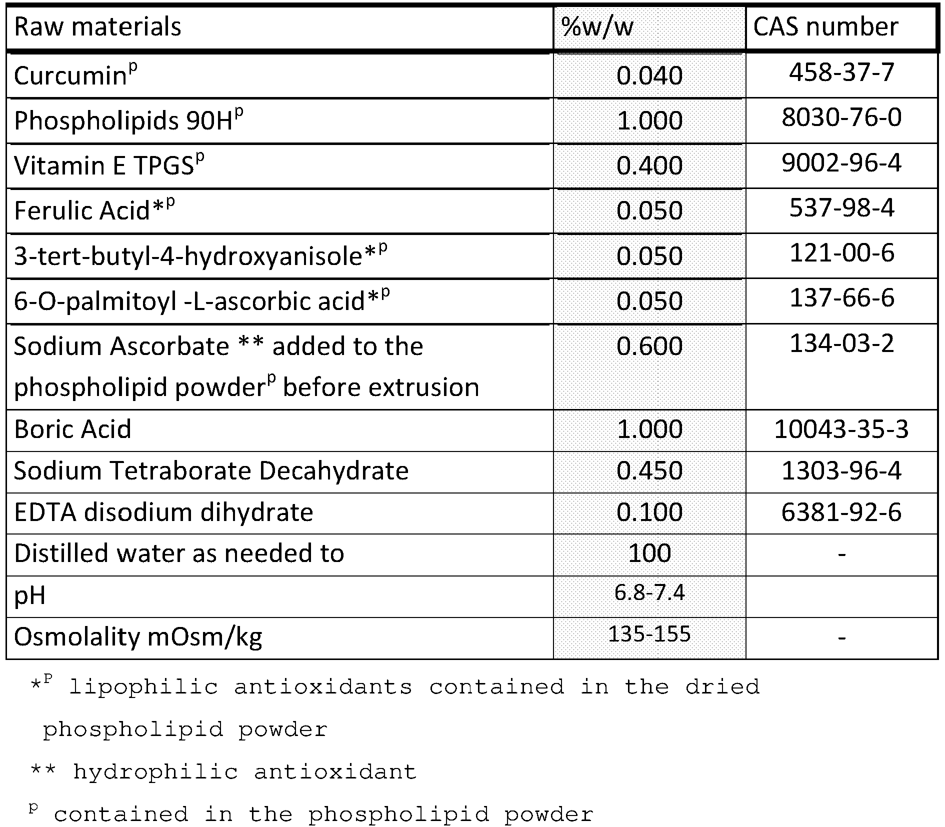

Once the extrusion was finished, and the appropriate controls, pH and osmolality were carried out, other Sodium Ascorbate was added to the liposomal solution to reach a concentration of 1.2%, so as to enhance the protection of the Curcumin Liposomes in the subsequent 0.2µ Filtration step and stability over time.

### Formulation NLL-191:

### Curcumin Liposomes (Vitamin E TPGS 0.4% + Sodium Ascorbate (0.6+0.6=1.2%), Preparation of 50 liters, use of a 20 inch filter (0.8-0.2 micron) (FILTRATION RATE)

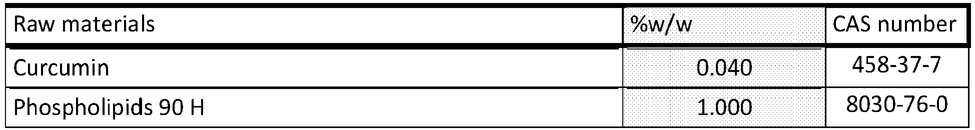

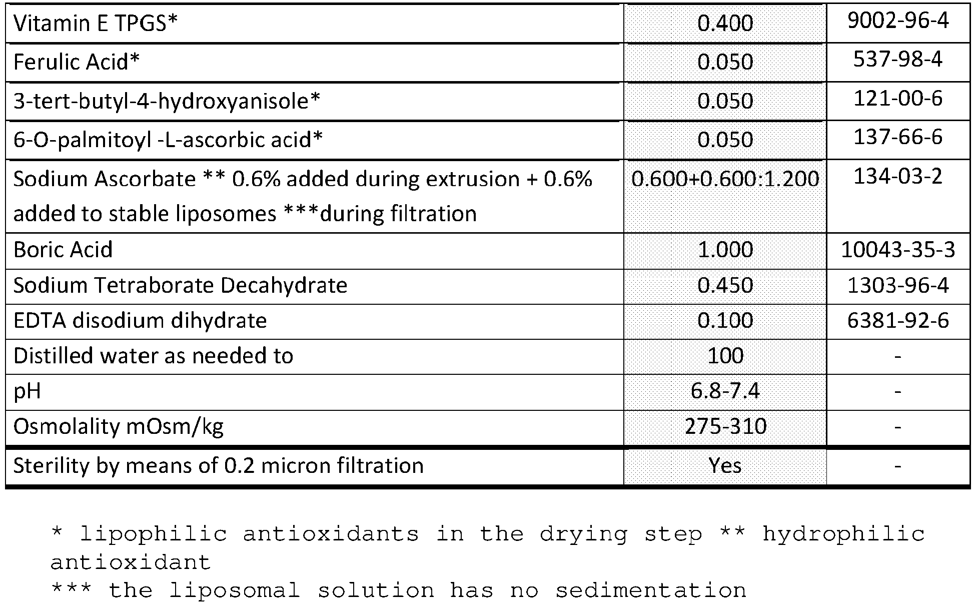

| PREPARATION DATA | % | *Loss % |
|---|---|---|
| Curcumin theoretical value before extrusion | 0.040 | |
| Titer of Curcumin in liposomes after extrusion and filtration | 0.0242 | 39.5 |

| | | |
|---|---|---|
| *Loss % with respect to theoretical | | |

### - Preparation data review

The titer of curcumin after extrusion and filtration is 0.0242% with a decrease of 39.5% with respect to the theoretical titer of 0.04%.

The result is lower than the previous formulation consisting of non-hydrogenated phospholipids, while having the same concentration of all constituents (lipophilic antioxidants) during the drying step, including vitamin E TPGS and the same final concentration of sodium ascorbate.

This datum indicates that:
the liposomes with hydrogenated phospholipids are more difficult to filter,
the curcumin is intercalated in the phospholipids, and
the concentration of 0.4% of vitamin E TPGS does not affect the solubilization of curcumin or does not solubilize the same phospholipids (if so, we would have the same curcumin titer), but serves to make the phospholipids fluid, especially the non-hydrogenated phospholipids.

### STABILITY OF THE FORMULATION NLL-191

The above Formulation NLL-191 was stabilized, as shown in fig. 2.

### Stability 4°C,25°C,40°C

| Time months | %w/w 4°C | Degr% | %w/w 25°C | Degr% | %w/w 40°C | Degr % |
|---|---|---|---|---|---|---|
| To | 0.0242 | 0 | 0.0242 | 0 | 0.0242 | 0 |
| T1 | 0.0239 | 1.24 | 0.0235 | 2.9 | 0.0234 | 3.303 |
| T2 | 0.0235 | 2.9 | 0.0232 | 4.13 | 0.0224 | 7.44 |
| T3 | 0.0229 | 5.37 | 0.0225 | 7.3 | 0.0219 | 9.55 |
| T4 | 0.0227 | 6.2 | 0.0222 | 8.27 | 0.0214 | 11.58 |
| T5 | 0.0225 | 7.03 | 0.0214 | 11.58 | 0.0206 | 14.77 |
| T6 | 0.0224 | 7.44 | 0.0213 | 11.99 | 0.0204 | 15.70 |
| Degradation % | | 7.44 | | 11.99 | | 15.70 |

### Review of stability data

The presence of Ferulic acid, BHA, Vitamin E TPGS and Sodium Ascorbate led to a significant improvement in the stability of curcumin, at all temperatures 4°C, 25°C and 40°C.

In particular, the result at "6 months at 40°C" is comparable to that at "24 months at room temperature".

Conclusion: the preparation of curcumin liposomes must take into account the stabilization of curcumin during the drying step: the particular antioxidants/Lipophilic anti-radicals in this step contribute to the final stability.

The addition of 0.6% Sodium Ascorbate outside the liposomes consisting of Hydrogenated Phospholipids during extrusion and then always at 0.6% during the filtration step does not particularly affect the final titer of the final product: in fact, the final titer with respect to the theoretical titer decreases by 39.5%, while overall the stabilization of Curcumin during the stability test is always very effective.

In short, the hydrogenated phospholipids, of which the NLL191 formulation consists, are much more rigid than the non-hydrogenated phospholipids, do not substantially improve the stability of Curcumin over time, but probably make it more difficult to filter the liposomes themselves even in the presence of Vitamin E TPGS at the same concentration present in the formulation NLL 190 with less rigid non-hydrogenated phospholipids.

In fact, the titer of curcumin is 0.0242% with respect to 0.0295% of the formulation (NLL 190) of liposomes with the non-hydrogenated phospholipids and with the ascorbic acid added only to the already formed liposomes.

### Final consideration:

The data reported for the formulations NLL 190 and NLL 191 give a precise indication of how the strategy of adding lipophilic antioxidants in the drying step is important to obtain a stable final formulation, but this protection is a necessary but insufficient condition because it must be associated with an agent, sodium ascorbate, having the ability to eliminate dissolved oxygen in solution.

The addition of sodium ascorbate can be done to the solution containing the already formed liposomes (see formulation 190), obtaining a product stable for 6 months at 40°C, corresponding to 24 months at room temperature, with a % degradation of 14.58%.

On the other hand, the addition of sodium ascorbate can be done in a fractional manner, a first aliquot during the extrusion of the phospholipid powder and a second aliquot with the liposomes just formed, obtaining a formulation (NLL 191) which is also stable over time for 6 months at 40°C, corresponding to 24 months at room temperature, equal to 15.70%, although slightly lower than the formulation NLL 190, 15.70% versus 14.58.

In particular, the degradation of curcumin in an aqueous buffer at physiological pH is an auto-oxidation, which is a direct consequence of the antioxidant action thereof.

The trend (the chain reaction) of the radical reaction leads to the stable incorporation of oxygen (dissolved in the medium) by curcumin with the resulting formation of a degradation product, the dioxygenated Bicyclopentadione. (1a)

At this point, the choice of using sodium ascorbate, rather than ascorbic acid, must be clarified precisely. Such a choice arises from the fact that the ascorbate anion is the predominant species at biological pH values (6.8-7.4), which are those prescribed for eye drops in association with osmolality (270-310 mOsM/kg).

It is a reducing agent (not too strong, mild) and an antioxidant, which oxidizes with the loss of a first electron, with the formation of a "radical anion", which then, with the loss of a second electron, forms dehydroascorbic acid, as shown in fig. 3.(See ref. 4a). At this point, having unequivocally defined the stability of the two liposomal preparations over time, with hydrogenated and non-hydrogenated phospholipids, the inventors undertook to evaluate the possibility of a faster production of liposomes containing Curcumin, which only included the extrusion step, and the subsequent 0.2 micron filtration necessary for the sterilization of the liposomes.

A formulation FAST and the production method thereof, that we report below, were developed:

### Method: Formulation Fast A

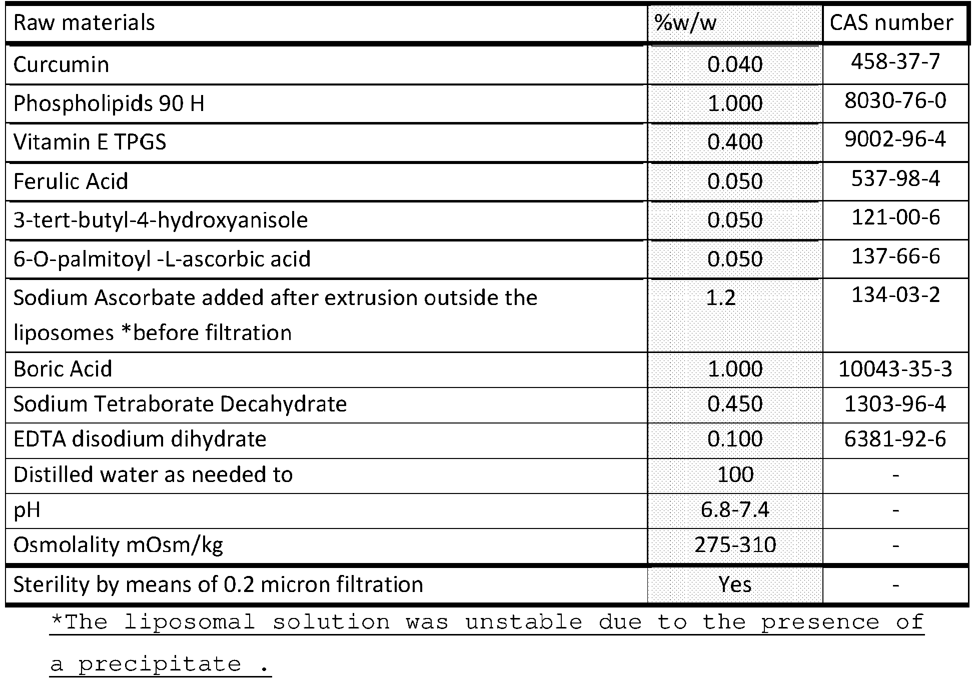

Formulation Fast A, comprising:
a) mixing all the constituents of the formulation; curcumin, non-hydrogenated phospholipids, Vitamin E TPGS, ferulic acid, 3-tert-butyl-4-hydroxyanisole*, 6-O-palmitoyl -L-ascorbic acid
b) Repeated extrusion of the dispersion to obtain the liposomes;
c) addition of Sodium Ascorbate to the liposomes;
d) sterilization of the liposomal solution with ascorbate by means of 0.2 micron filtration.

### FAST A Results.

| Initial theoretical concentration % | After filtration |
|---|---|
| 0.040 | 0.0216 |
| Reduction % with respect to the initial theoretical value | 0.0216/0.040x100 = 46 |

### FAST A Results.

| Concentration % after filtration | After 1 month at 40°C |
|---|---|
| 0.0216 | 0.0178 |
| Decrease % with respect to the value after filtration | 0.0178/0.0216x100 =17.7 |

Conclusions: The suggested procedure which reports the direct extrusion of the constituents of the formulation, (Curcumin, Phospholipids S80, Vitamin E TPGS, Ferulic Acid, 3-tert-butyl-4-hydroxyanisole, 6-O-palmitoyl -L-ascorbic acid + Ascorbate Acid) produces unstable liposomes which tend to precipitate in time (see photo no. 4). In part, this peculiarity justifies the lower concentration of 0.0216% with respect to 0.0295% obtained from the formulation NLL 190 and 0.0242% obtained from the formulation NLL 191.

Moreover, the decrease after 1 month of stability at 40°C is equal to 17.7% with respect to the initial value after filtration, while for the formulation NLL 190, after 1 month of stability at 40°C it is equal to 8.04% and after 6 months of stability it is 14.58%, for the formulation NLL 191, after 1 month of stability at 40°C it is equal to 3.303 and after 6 months of stability at 40°C it is equal to 15.70%.

This result indicates a lower capacity of the liposomal structure to intercalate the curcumin molecules and therefore, since it is less inside the phospholipids, is more exposed to the degrading action of oxygen.

### REFERENCES

1) Journal of Pharmaceutical and Biomedical Analysis Volume 15, Issue 12, August 1997, Pages 1867-1876
   Stability of curcumin in buffer solutions and characterization of its degradation products
1a) Trends Mol Med. 2012 Jul; 18(7): 361-364. Vanillin and ferulic acid are not the major degradation products of curcumin Odaine N. Gordon and Claus Schneider
2) Biochem Pharmacol. 1990 May 15;39(10):1597-601. Comparative evaluation of the antioxidant activity of alpha-tocopherol, alpha-tocopherol polyethylene glycol 1000 succinate and alpha-tocopherol succinate in isolated hepatocytes and liver microsomal suspensions. R Carini 1, G Poli, M U Dianzani, S P Maddix, T F Slater, K H Cheeseman
3) J. Agric. Food Chem. 2002, 50, 7, 2161-2168 Antioxidant Properties of Ferulic Acid and Its Related Compounds Hiroe KikuzakiMasashi HisamotoKanae HiroseKayo Akiyamaand Hisaji Taniguchi
4) J Agric Food Chem. 2015 Feb 18;63(6):1819-24. How the multiple antioxidant properties of ascorbic acid affect lipid oxidation in oil-in-water emulsions Sibel Uluata 1, D Julian McClements, Eric A Decker
4b) Ascorbic Acid-Based Oxygen Scavenger in Active Food Packaging System for Raw Meatloaf Jung-Soo Lee, Yoonjee Chang, Eun-Sil Lee, Hong-Geon Song, Pahn-Shick Chang, and Jaejoon Han 5) 2016 Mar;60(3) :487-94.Epub 2015 Dec 18.Redox modulation of curcumin stability: Redox active antioxidants increase chemical stability of curcumin Yoshiki Nimiya 1 2, Weicang Wang 1, Zheyuan u 1, Elvira Sukamtoh 1, Julia Zhu 1, Eric Decker 1, Guodong Zhang ¹
6) Ter ButylAnysole BHA: US Patent 10 279 005 B2, Gore et All.
7) 2016 Mar;60(3) :487-94.Epub 2015 Dec 18. Redox modulation of curcumin stability: Redox active antioxidants increase chemical stability of curcumin Yoshiki Nimiya 1 2, Weicang Wang 1, Zheyuan Du 1 Elvira Sukamtoh 1, Julia Zhu 1, Eric Decker 1, Guodong Zhang ¹

## Claims

1. A method for preparing a liposomal formulation of curcumin for ophthalmic use, consisting of extruded curcumin liposomes, wherein the amount of curcumin ranges from 0.025% to 0.050% by weight, on the total weight, and wherein vitamin E-D-α- polyethylene glycol (E-TPGS) is further present in an amount between 0.05 and 0.5% by weight, with respect to the weight of the final composition, **characterized in that**, to improve the stability of the product obtained,
before the vacuum drying step, to the starting alcoholic solution containing phospholipids, curcumin, and vitamin E TPGS there are added:
antioxidants/anti-lipophilic radicals, such as BHA and ascorbyl palmitate, capable of directly interacting with phospholipids and curcumin; and
ferulic acid, capable of inhibiting /slowing down, at the chemical equilibrium level, the hydrolysis reaction of the curcumin itself,
while
before the 0.2 micron filtration step, sodium ascorbate is added to the aqueous solution of freshly extruded liposomes, which, outside the liposomes, removes oxygen dissolved in the liposomal solution, thus increasing the stabilization of the curcumin itself.

2. The method for preparing a liposomal formulation of curcumin for ophthalmic use, according to claim 1, **characterized in that** the phospholipids are hydrogenated.

3. The method for preparing a liposomal formulation of curcumin for ophthalmic use, according to claim 1, **characterized in that** the phospholipids are not hydrogenated.

4. The method for preparing a liposomal formulation of curcumin for ophthalmic use, according to claim 1, **characterized in that** it includes adding an antioxidant such as sodium ascorbate at a concentration by weight of 1.2% of the total weight of the formulation.

5. The method for preparing a liposomal formulation of curcumin for ophthalmic use, according to claim 4, **characterized in that** sodium ascorbate is added in a percentage of 0.6% by weight in the aqueous phase before extrusion and in an equal amount of 0.6% by weight on the total weight of the formulation added before filtration.

## Patentansprüche

1. Verfahren zum Herstellen einer liposomalen Formulierung von Curcumin zur ophthalmischen Verwendung, umfassend extrudierte Curcumin-Liposomen, wobei die Menge an Curcumin im Bereich von 0,025 Gew.-% bis 0,050 Gew.-%, bezogen auf das Gesamtgewicht, liegt und wobei Vitamin E-D-α-Polyethylenglykol (E-TPGS) ferner in einer Menge zwischen 0,05 und 0,5 Gew.-%, bezogen auf das Gewicht der endgültigen Zusammensetzung, vorhanden ist, **dadurch gekennzeichnet, dass** zur Verbesserung der Stabilität des erhaltenen Produkts
vor dem Schritt des Vakuumtrocknens zu der die Phospholipide, Curcumin und Vitamin E TPGS enthaltenden alkoholischen Ausgangslösung zugesetzt werden:
Antioxidantien/antilipophile Radikale, wie BHA und Ascorbylpalmitat, die in der Lage sind, direkt mit den Phospholipiden und dem Curcumin zu interagieren; und
Ferulasäure, die in der Lage ist, auf dem Niveau des chemischen Gleichgewichts die Hydrolysereaktion des Curcumins selbst zu hemmen oder zu verlangsamen,
wobei ferner
vor dem 0,2-µm-Filtrationsschritt der wässrigen Lösung frisch extrudierter Liposomen Natriumascorbat zugesetzt wird, welches außerhalb der Liposomen den in der liposomalen Lösung gelösten Sauerstoff entfernt, wodurch die Stabilisierung des Curcumins selbst erhöht wird.

2. Verfahren zum Herstellen einer liposomalen Formulierung von Curcumin zur ophthalmischen Verwendung nach Anspruch 1, **dadurch gekennzeichnet, dass** die Phospholipide hydriert sind.

3. Verfahren zum Herstellen einer liposomalen Formulierung von Curcumin zur ophthalmischen Verwendung gemäß Anspruch 1, **dadurch gekennzeichnet, dass** die Phospholipide nicht hydriert sind.

4. Verfahren zum Herstellen einer liposomalen Formulierung von Curcumin zur ophthalmischen Verwendung gemäß Anspruch 1, **dadurch gekennzeichnet, dass** es das Zusetzen eines Antioxidans wie Natriumascorbat in einer Gewichtskonzentration von 1,2 % des Gesamtgewichts der Formulierung umfasst.

5. Verfahren zum Herstellen einer liposomalen Formulierung von Curcumin zur ophthalmischen Verwendung gemäß Anspruch 4, **dadurch gekennzeichnet, dass** Natriumascorbat in einem Prozentsatz von 0,6 Gew.-% in der wässrigen Phase vor der Extrusion und in einer gleichen Menge von 0,6 Gew.-%, bezogen auf das Gesamtgewicht der Formulierung, vor der Filtration zugesetzt wird.

## Revendications

1. Procédé pour préparer une formulation liposomale de curcumine pour une utilisation ophtalmique, consistant en des liposomes de curcumine extrudés, dans lequel la quantité de curcumine varie de 0,025 % à 0,050 % en poids, sur le poids total, et dans lequel la vitamine E-D-α-polyéthylène glycol (E-TPGS) est en outre présente en une quantité comprise entre 0,05 et 0,5 % en poids, par rapport au poids de la composition finale, **caractérisé en ce que**, pour améliorer la stabilité du produit obtenu, avant l'étape de séchage sous vide, à la solution alcoolique de départ contenant des phospholipides, de la curcumine et de la vitamine E TPGS sont ajoutés :
des antioxydants/radicaux antilipophiles, tels que le BHA et le palmitate d'ascorbyle, capables d'interagir directement avec les phospholipides et la curcumine ; et
l'acide férulique, capable d'inhiber/de ralentir, au niveau de l'équilibre chimique, la réaction d'hydrolyse de la curcumine elle-même,
pendant que
avant l'étape de filtration à 0,2 micron, de l'ascorbate de sodium est ajouté à la solution aqueuse de liposomes fraîchement extrudés, qui, en dehors des liposomes, élimine l'oxygène dissous dans la solution liposomale, augmentant ainsi la stabilisation de la curcumine elle-même.

2. Procédé pour préparer une formulation liposomale de curcumine pour une utilisation ophtalmique, selon la revendication 1, **caractérisé en ce que** les phospholipides sont hydrogénés.

3. Procédé pour préparer une formulation liposomale de curcumine pour une utilisation ophtalmique, selon la revendication 1, **caractérisé en ce que** les phospholipides ne sont pas hydrogénés.

4. Procédé pour préparer une formulation liposomale de curcumine pour une utilisation ophtalmique, selon la revendication 1, **caractérisé en ce qu'**il inclut l'ajout d'un antioxydant tel que l'ascorbate de sodium à une concentration en poids de 1,2 % du poids total de la formulation.

5. Procédé pour préparer une formulation liposomale de curcumine pour une utilisation ophtalmique, selon la revendication 4, **caractérisé en ce que** l'ascorbate de sodium est ajouté en pourcentage de 0. 6 % en poids en phase aqueuse avant extrusion et en quantité égale à 0,6 % en poids sur le poids total de la formulation ajoutée avant filtration.
